# EUROPEAN PATENT APPLICATION

(11) **EP 0 846 772 A1**
(43) Date of publication of application: **10.06.1998**
(21) Application number: 97120939.0
(22) Date of filing: 28.11.1997
(51) Int. Cl.: C12N 15/87, C07K 16/28, C12N 15/62, C07K 16/44, A61K 48/00, C12N 9/24

(54) **Multifunctional ligand system for cell-specific transfer of nucleic acid**

(30) Priority: 29.11.1996 DE 19649645
(71) Applicant: HOECHST AKTIENGESELLSCHAFT, 65929 Frankfurt am Main (DE)
(72) Inventor: Sedlacek, Hans-Harald, Prof. Dr., 35041 Marburg (DE); Müller, Rolf, Prof. Dr., 35037 Marburg (DE)
(74) Representative: Bösl, Raphael, Dr. rer. nat., Dipl.-Ing.

(57) **Abstract**

The invention relates to a multifunctional ligand system that is not immunogenic and that carries out cell-specific transfer of nucleotide sequences. The system comprises at least one target cell-specific ligand, at least one linker and at least one gene construct-specific ligand, with the gene construct-specific ligand advantageously comprising an antibody, or a part thereof, which binds directly or indirectly to the gene construct. The invention also relates to ligand systems for preparing a pharmaceutical or a vaccine for the treatment or prevention of a disease of the skin, of the mucous membranes, of the nervous system, of the internal organs, of blood coagulation, of the hemopoietic system, of the immune system, of the musculature, of the sustentacular tissue or of the joints.

## Description

### Field of the Invention

The invention relates to improved procedures and agents for transfering polynucleotide into cells.

### Background of the Invention

The binding of a gene construct to a cell surface is a necessary, even if not sufficient, prerequisite for transferring genes into a cell. The stronger this binding, the greater the probability is that the gene construct will be successfully taken up by the cell and transcribed in the cell. The more cell-specific this binding, the more likely it is that the gene construct will bind predominantly or solely to the target cell and be taken up by this cell. The specificity of the binding of a gene construct to the target cell is of particular importance when cells of another type are present in addition to the gene construct and the target cell but the gene construct has nevertheless to be taken up preferentially or exclusively by the target cell.

Various technologies have been developed that enable a gene construct to bind in a cell-specific manner. It is common to all these technologies that they make use of the binding of (1) a ligand to its receptor which is expressed on the cell membrane, or (2) an antibody to its antigen or hapten which is exposed on the cell membrane. Examples of such technologies include recombinant methods to incorporate ligands such as heregulin (Han et al., PNAS 92, 9747 (1995)), erythropoietin (Kasahara et al., Science 266, 1373 (1994)), antibody fragments such as single-chain Fv (Marin et al., J. Virol 70, 2957 (1996)) or receptors such as the extracellular moiety of the Fc receptor (Dougherty et al., Transfusion Science 17, 121 (1996)) into the coat glycoprotein of retroviral vectors and bring about target cell specificity in this manner.

Chemical methods also have been used to link ligands such as asialoglycoprotein (Wu et al., J. Biol. Chem. 269, 1152 (1994)) or synthetic derivatives of this protein (Marwin et al., Bioconjugate Chem. 5, 612 (1994)) to polylysine and either complex the latter with the gene construct or bind it chemically to coat proteins of adenoviral vectors. Another method is to bind ligands to streptavidin, which in turn binds to biotin which is conjugated to the phospholipid head groups of liposomes, with the liposomes being complexed with gene constructs (Redelmeir et al., Drug Deliv. J. Deliv. Targeting Therap. Agents 2, 98, (1995)). A first ligand system was presented by Fominaya et al., J. Biol. Chem 271, 10560, 1996. This ligand system comprises an antibody fragment which is specific for the Erb B2 receptor on tumor cells, the Pseudomonas fusogenic peptide exotoxin A and the DNA-binding domain of the yeast Gal-4 protein, which binds to the corresponding Gal-4 binding sequence which is inserted into a plasmid containing the transgene. While this ligand system results in target cell-specific transfection, it suffers from the disadvantage of the immunogenicity of the yeast Gal-4 protein.

None of the these published methods adequately solved the problem of binding vectors in a target cell-specific manner. The main reasons for this lack of specificity include: (1) impairment of the function of the modified viral vectors; (2) the considerable complexity and size of the ligand systems used; (3) the immunogenicity and compatibility of heterologous or modified proteins, or of the streptavidin-biotin coupling system employed; and (4) an inadequate ability of the bound gene construct to effect target cell-specific transduction of cells.

As a result of these limitations, a great need remains for a simply prepared, functionally capable ligand useful for binding viral and nonviral vectors to target cells.

### Summary of the Invention

Accordingly, it is an object of the invention to provide a ligand system for target cell-specific transfer of nucleotide sequences that is not immunogenic and that is simpler to prepare and use than previously known systems. Another object is to increase the specificity of binding vectors to target cells, and thereby improve the efficiency and selectivity of transfering nucleic acid into target cells.

In accomplishing these and other objectives, one aspect of this invention provides a multifunctional ligand system for target cell-specific transfer of nucleotide sequences, comprising at least one target cell-specific ligand, at least one gene construct-specific ligand that comprises an antibody or an antibody portion, and a linker that links the two ligands, wherein the system is not immunogenic. In an advantageous embodiment at least one ligand is a humanized antibody or antibody fragment. In another advantageous embodiment a composition is provided for curing a disease, comprising a multifunctional ligand system for target cell-specific transfer of nucleotide sequences, comprising at least one target cell-specific ligand, at least one gene construct-specific ligand that comprises an antibody or an antibody portion, and a linker that links the two ligands, wherein the system is not immunogenic and is provided in a suitable carrier for administration to a patient.

In another advantageous embodiment the ligand system comprises a connector that connects two ligands together and the connector comprises two linkers. In another advantageous embodiment a ligand system is provided that comprises: (a) a TS ligand comprising an anti-NCAM recombinant single-chain Fv fragment, wherein the variable heavy chain and light chain of the Fv fragment are covalently linked by way of a short peptide sequence; (b) a linker comprising a fusogenic peptide having the sequence GLFEALLELLESLWELLLEA (SEQ ID No.: 1); and (c) a GS ligand comprising a recombinant antibody for N⁶⁻methyladenine.

In another advantageous embodiment, the invention concerns the use of a ligand system according to the present invention for the manufacture of a pharmaceutical to prevent or treat a skin disease, mucous membrane disease, nervous system disease, internal organ disease, blood coagulation disease, hematopoietic system disease, immune system disease, musculature disease, and sustentacular tissue or joint disease. The ligand system is locally administered or injected into a patient or, alternatively, to cells obtained from a patient to prevent, as a vaccine, or to treat a disease associated with one or more of the group consisting of skin, mucous membrane, nervous system, internal organs, blood coagulation, hematopoietic system, immune system, musculature, sustentacular tissue and joints.

These advantages are made possible by linking cell specific ligands to gene constructs in accordance with the invention.

Other objects, features and advantages of the present invention will become apparent from the following detailed description. It should be understood, however, that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

Figure 1 shows three alternative ligand systems composed of target cell-specific ligands and gene construct-specific ligands in which the ligands are linked by a linker (Figure 1a and 1b) or by a "connector" (Figure 1c).

Figure 2 presents an embodiment of the ligand system of Figure 1c wherein the "connector" comprises the hinge region of an antibody.

Figure 3 displays another embodiment of the ligand system of Figure 1c wherein the "connector" comprises a Gal80 protein and the Gal80-binding domain of Gal4.

Figure 4 depicts an embodiment wherein a fusogenic peptide is used to link two antibody fragments.

Figure 5 schematicizes expression vectors pHENIS and pAB1, which are useful in the invention.

### Detailed Description of the Preferred Embodiments

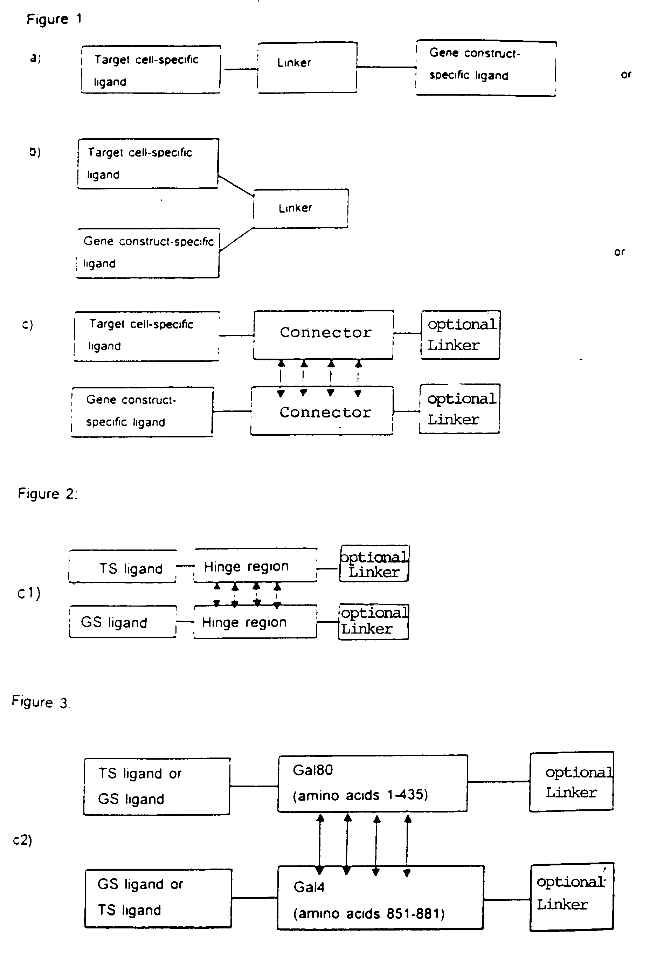

The present inventors discovered that a target cell-specific ligand and a gene construct-specific ligand could be connected to each other, without forming an immunogenic complex, by a linker as shown in Figure 1. Furthermore, by using, in particular, a "connector" as shown in Figure 1c and Figures 2 and 3, a ligand system could be constructed to overcome prior art limitations of immunogenicity, poor specificity and efficiency of transduced cells.

A "target cell-specific ligand" (TS ligand) as termed herein is a molecule which binds to a determinant on the surface of a target cell, i.e. to a binding partner of the target cell-specific ligand, for example to a receptor or to an adhesion molecule.

A "linker" is a molecule which, in the simplest case, links the target cell-specific ligand with the gene construct-specific ligand and advantageously may possess fusogenic properties, i.e. properties which permit penetration of the novel ligand system through the cell membrane and/or through the lysosomal membrane, i.e. from the lysosomes into the cytoplasm. In an advantageous embodiment of the invention, the linker also has fusogenic properties.

A "gene construct-specific ligand" (GS ligand) is a molecule which binds directly or indirectly, by way of an antibody or a part thereof, to a gene construct.

In a suitable embodiment of the invention, the TS ligand and the GS ligand are linked by a "connector." In addition, either or both ligands may contain separate linkers. Embodiments that utilize a connector can assume different forms depending on the specific GS ligand and TS ligand used (e.g. 1 × linker, 2 × TS ligand, 1 × GS ligand or 1 × linker, 1 × TS ligand, 2 × GS ligand).

Coupling between the TS ligand, the linker and the GS ligand can be effected by covalent bonding [description of the technology is given by Sedlacek et al. Contrib. to Oncol. 32, 42 - 49 and 81 - 85, Karger Verlag Munich (1988)] or by non-covalent means such as that based on differing charges (ionic bonding). However, the ligand system also can be prepared as a fusion protein using recombinant techniques, as has already been described in EP-A1-0 464 533.

The nucleic acid sequence that is introduced into a targeted cell by means of the inventive ligand system can be a naked RNA or DNA, a naked RNA or a naked DNA mixed with a nonviral carrier, or a virus. During use, a ligand system of the invention is mixed with the gene construct and the resulting complex is added to cells that are to be transduced or, the complex may be administered to the patient.

Examples of useful target cell-specific ligands, gene construct-specific ligands, linkers and connectores for the invention are listed here to illustrate some of the possible combinations contemplated by the inventors.

### THE TS LIGAND

Within the context of the present invention, the TS ligand can be an active compound, a part of the active compound or an analog of the active compound which binds to a receptor on the target cell. Endogenous substances, portions of endogenous substances, and other substances that mimic one or more epitopes of an endogenous substance are advantageous because of their low immunogenicity, compared with most foreign substances when introduced into a body.

Examples of such active compounds are: growth factors such as VEGF, PDGF, EGF, TGFα, TGFβ, KGF, SDGF, FGF, IGF, HGF, NGF, BDNF, neurotrophins, BMF, bombesins, M-CSF, thrombopoietin, erythropoietin, SCF, SDGF, oncostatin, PDEGF and endothelin 1; cytokines such as IL-1, IL-2, IL-3, IL-4, IL-5, IL-6, IL-7, IL-8, IL-9, IL-10, IL-11, IL-12, IL-13, IL-14 and IL-15; interferon α, β and γ; tumor necrosis factors TNFα and TNFβ; chemokines such as RANTES, MCAF, MIP-1α, MIP-1β, NAP and β-thromboglobulin; peptide hormones such as SRH, SIH, STH, MRH, MSH, PRH, PIH, prolactin, LH-RH, FSH-RH, LH/ICSH, FSH, TRH, TSH, CRH, ACTH; angiotensin, a kinin, or histamine or homologs or analogs thereof; steroid hormones such as estrogens, gestagens, androgens, glucocorticoids or mineralocorticoids, or homologs or analogs thereof; and vitamins such as folic acid.

Within the context of the present invention the TS ligand also can be an adhesion molecule, a part of an adhesion molecule or an analog of an adhesion molecule which binds to a corresponding adhesion molecule which is located in the cell membrane or to another structure of the target cell which specifically binds an adhesion molecule.

Examples of such adhesion molecules which are capable of functioning as TS ligands are Lewis X (for GMP-140),S-Lewis X (for ELAM-1), LFA-1 (for ICAM-1 and ICAM-2), MAC-1 (for ICAM-1), VLA-4 (for VCAM-1), PECAM (for PECAM), vitronectin (for the vitronectin receptor), GMP-140 (for Lewis X), S-Lewis X (for ELAM-1), ICAM-1, ICAM-2 (for LFA-1, MAC-1), VCAM-1 (or VLA-4), fibronectin (for VLA-4), laminin (for VLA-6), fibronectin, laminin (for VLA-1, VLA-2, VLA-3), fibronectin (for VLA-4), fibrinogen (for GPIIb-IIIa), B7 (for CD28), CD28 (for B7), CD40 (for CD40L) and CD40L (for CD40).

Within the context of the present invention, the TS ligand also can be the extracellular part of an Fc receptor (Dougherty et al., Transfusion Science 17, 121 (1996)), to which an antibody which is specific for the target cell is bound by way of its Fc moiety.

Within the context of the present invention, the TS ligand also can be a ligand for a VDL receptor or an LDL receptor (e.g. LDL receptor, LDL-related receptor protein, FC-receptor of IgG (e.g. Fcγ RII-B2, Fcγ RI); 88 kDa glycoprotein receptor, acetylated LDL receptor, oxidized LDL receptor or megalin). Ligands of this nature have already been described in detail in the literature and are, for example: apolipoprotein B-100 or fragments thereof containing the carboxyterminal moiety, apolipoprotein E, protease/inhibitor complex, such as the tPA/PAI-1 complex, 2-macroglobulin, thrombospondin or its heparin-binding aminoterminal domain (e.g. amino acids 1-214), oxidized LDL, acetylated LDL or acetoacetylated LDL, acetylated lysine, desialylated LDL, malondialdehyde-conjugated LDL, formaldehyde-treated or glutaraldehydetreated albumin, maleylated albumin, 39 kDa receptor-associated protein, or fragments thereof, for example those containing the amino acids 18-112, 113-218 and 219-323 or the amino acids 1-114 and 114-319, lactoferrin, aprotinin, lipoprotein lipase, amyloid precursor protein (protease nexin 2) and Pseudomonas exotoxin.

Within the context of the present invention, the TS ligand also can be an antibody molecule or the epitope-binding moiety of an antibody molecule.

A murine monoclonal antibody, if used, preferably is employed in a humanized form to limit its immunogenicity. The humanization can be effected in the manner described by Winter et al. (Nature 349, 293 (1991)) and Hoogenboom et al. (Rev. Tr. Transfus. Hemobiol. 36, 19 (1993)). Antibody fragments are prepared in accordance with the state of the art, for example in the manner described by Winter et al., Nature 349, 293 (1991), Hoogenboom et al. Rev. Tr. Transfus. Hemobiol. 36, 19 (1993), Girol, Mol. Immunol. 28, 1379 (1991) or Huston et al., Int. Rev. Immunol. 10, 195 (1993).

Recombinant antibody fragments can be prepared directly from existing hybridomas or can be isolated from libraries of murine or human antibody fragments using the phage-display technology (Smith, Science 228, 1315 (1985)) (Winter et al., Annu. Rev. Immunol. 12, 433 (1994)). These antibody fragments are then used directly at the genetic level for further manipulations (e.g. fusion with other proteins).

In order to prepare recombinant antibody fragments from hybridomas, the genetic information which encodes the antigen-binding domains (VH, VL) of the antibodies is obtained by isolation of the mRNA, reverse transcription of the RNA in cDNA and subsequent amplification, using the polymerase chain reaction (Saiki et al., Science 230, 1350 (1985)) and oligonucleotides which are complementary to the 5' and 3' ends, respectively, of the variable fragments (Orlandi et al., PNAS-USA 86, 3833 (1989)). The VH and VL fragments are then cloned into bacterial expression vectors, for example in the form of Fv fragments (Skerra & Plückthun, Science 240, 1038 (1988)), single-chain Fv fragments (sc-Fc) (Bird et al., Science 242, 423 (1988), Huston et al., PNAS-USA 85, 5879 (1988)) or as Fab fragments (Better et al., Science 240, 1041 (1988)).

New antibody fragments also can be isolated directly from antibody libraries (immune libraries, naive libraries) of murine or human origin using the phage-display technology. In the phage display of antibody fragments, the antigen-binding domains are cloned, as fusion proteins with the g3P coat protein of filamentous bacteriophages, either into the phage genome (McCafferty et al., Nature 348, 552 (1990)) or into phagemid vectors (Breitling et al., Gene 104, 147 (1991)) in the form of scFv fragments (McCafferty et al., Nature 348, 552 (1990)) or as Fab fragments (Hoogenboom et al., Nucl. Acid Res. 19, 4133 (1991), Barbas et al., PNAS-USA 88. 7978 (1991)). Antigen-binding phages are selected on antigen-loaded plastic vessels (panning) (Marks et al., J. Mol. Biol. 222, 581 (1991)), on antigen-conjugated paramagnetic beads (Hawkins et al., J. Mol. Biol. 226, 889 (1992)) or by binding to cell surfaces (Marks et al., Bio/Technol. 11, 1145 (1993)).

Immune libraries can be prepared by PCR amplification of the variable antibody fragments from the B lymphocytes of immunized animals (Sastry et al., PNAS-USA 86, 5728 (1989), Ward et al., Nature 341, 544 (1989), Clackson et al., Nature 352, 624 (1991)) or patients (Mullinax et al., PNAS-USA 87, 8095 (1990), Barbas et al., PNAS-USA 88, 7978 (1991)). For this, use is made of combinations of oligonucleotides which are specific for murine (Orlandi et al., PNAS-USA 86, 3833 (1989), Sastry et al., PNAS-USA 86, 5728 (1989), or human immunoglobulin genes (Larrick et al., BBRC 160, 1250 (1989)) or for the human immunoglobulin gene families (Marks et al., Eur. J. Immunol. 21, 985 (1991)).

Native gene libraries can be prepared by using non-immunized donors as the source of the immunoglobulin genes (Marks et al., J. Mol. Biol. 222, 581 (1991)). Alternatively, immunoglobulin germline genes can be used to prepare semisynthetic antibody repertoires, with the complementarity-determining region 3 of the variable fragments being amplified by PCR using degenerate primers (Hoogenboom & Winter, J. Mol. Biol. 227, 381 (1992), Barbas et al., PNAS-USA 89. 4457 (1992), Nissim et al., EMBO J. 13, 692 (1994), Griffiths et al., EMBO J. 13, 3245 (1994)). These so-called single-pot libraries have the advantage, as compared with immune libraries, that antibody fragments against a large number of antigens can be isolated from one single library (Nissim et al., EMBO J. 13, 692 (1994)).

The affinity of antibody fragments can be increased still further by using phage-display technology, with new libraries being prepared from already existing antibody fragments by means of random (Hawkins et al., J. Mol. Biol. 226, 889 (1992), Gram et al., PNAS-USA 89, 3576 (1992)), codon-based (Glaser et al., J. Immunol. 149, 3903 (1992)) or site-directed mutagenesis (Balint & Larrick, Gene 137, 109 (1993)), by the chain-shuffling of individual domains using fragments from naive repertoires (Marks et al., Bio/Technol. 10, 779 (1992)) or using bacterial mutator strains (Low et al., J. Mol. Biol. 260, 359 (1996)), and antibody fragments having improved properties being isolated by means of reselection under stringent conditions (Hawkins et al., J. Mol. Biol. 226, 889 (1992)). In addition, murine antibody fragments can be humanized by replacing one of the variable domains with a human repertoire in a step-wise manner and then selecting with the original antigen (guided selection) (Jespers et al., Bio/Technol. 12, 889 (1994)). Alternatively, murine antibodies can be humanized by specific replacement of the hypervariable regions of human antibodies with the corresponding regions of the original murine antibody (Jones et al., Nature 321, 522 (1987)).

Within the context of the present invention, the TS ligand also can be the envelope protein, or a part of the envelope protein, of viruses which specifically bind to selected cells by way of their envelope protein. The choice of the TS ligand depends on the target cell which is to be transduced with the gene construct.

Examples of ligands useful for the invention include substances that: activate endothelial cells, macrophages and lymphocytes; bind muscle cells, hematopoietic cells, synovial cells and inflammatory cells; bind cells that are infected with viruses; bind tissue cells such as liver cells; bind glia cells; and that bind leukemia cells. Some representative members of these ligands are summarized here.

### TS ligands for activated endothelial cells

Within the meaning of the invention, these ligands include antibodies or antibody fragments which are directed against membrane structures of endothelial cells, as have been described, for example, by Burrows et al. (Pharmac. Ther. 64, 155 (1994)), Hughes et al. (Cancer Res. 49, 6214 (1989)) and Maruyama et al. (PNAS-USA 87, 5744 (1990)). In particular, these ligands include antibodies against the VEGF receptors.

The TS ligands also include all active compounds which bind to membrane structures or membrane receptors on endothelial cells. For example, these ligands include substances which contain mannose in a terminal position, and, furthermore, IL-1 or growth factors, or their fragments or part sequences thereof, which bind to receptors which are expressed by endothelial cells, such as PDGF, bFGF, VEGF or TGFβ (Pusztain et al., J. Pathol. 169, 191 (1993)).

The TS ligands also include ligands for LDL receptors, for example for the acetylated LDL receptor, the oxidized LDL receptor, for the LDL-related receptor protein (LRP), for the 88 kDa glycoprotein receptor and for the Fc receptor for IgG. Ligands of this nature have already been described in detail in the literature.

The TS ligands furthermore include adhesion molecules which bind to activated and/or proliferating endothelial cells. Adhesion molecules of this nature, such as Slex, LFA-1, MAC-1, LECAM-1, VLA-4 or vitronectin, have already been described (reviews in Augustin-Voss et al., J. Cell. Biol. 119, 483 (1992), Pauli et al., Cancer Metast. Rev. 9, 175 (1990) and Honn et al., Cancer Metast. Rev. 11, 353 (1992)). Within the meaning of this invention, the TS ligands include, in particular, viral coat glycoproteins which possess a tropism for endothelial cells. Examples of these viruses are: filoviruses, for example Marburg virus with its GP (glycoprotein) and sGP (second glycoprotein) coat proteins or Ebola virus in each case with its GP and sGP coat proteins, cytomegalovirus in particular with its gB protein, herpes simplex virus type I, HIV-1 virus, measles virus, Hantaan virus, alphaviruses, such as Semliki Forest virus, epidemic hemorrhagic fever virus, polio virus, and an enteroviruses such as ECHO 9, ECHO 12 or Coxsackie B3.

### TS ligands for activated macrophages and/or activated lymphocytes.

Within the meaning of the invention, a ligand may include a substances that binds specifically to the surface of an immune cell. Such substances include antibodies or antibody fragments which are directed against membrane structures of immune cells, as have been described, for example, by Powelson et al., Biotech. Adv. 11, 725 (1993).

The TS ligands furthermore include all ligands for LDL receptors, as have already been described above. The TS ligands furthermore also include monoclonal or polyclonal antibodies or antibody fragments which bind, by their antigen-binding variable part, to Fc-γ or Fc-ε or Fc-µ receptors on immune cells (Rojanasakul et al., Pharm. Res. 11, 1731 (1994)).

These ligands also include the Fc fragment of human monoclonal or polyclonal immunoglobulin. Fc fragments of this nature are prepared, for example, by means of genetic manipulation using recombinant DNA or in accordance with the methods of Haupt et al., Klin. Wschr. 47, 270 (1969), Kranz et al., Dev. Biol. Standard 44, 19 (1979); Fehr et al., Adv. Clin. Pharmac. 6, 64 (1974), Menninger et al., Immunochem. 13, 633 (1976).

The TS ligands furthermore include all substances which bind to membrane receptors on the surface of immune cells. These ligands include cytokines, such as IL-1, IL-2, IL-3, IL-4, IL-6, IL-10, TNFα, GM-CSF and M-CSF, and also growth factors, such as EGF, TGF, FGF, IGF or PDGF, or their fragments or part sequences thereof, which bind to receptors which are expressed by immune cells.

The TS ligands furthermore include adhesion molecules and other ligands which bind to cell membrane structures, for example to the mannose 6-phosphate receptor on macrophages in spleen, liver, lung and other tissues. A selection of these ligands and membrane structures are reviewed in Perales et al., Eur. J. Biochem. 226, 255 (1994).

Within the meaning of this invention, the TS ligands also include envelope glycoproteins from those viruses which have a tropism for lymphocytes and/or macrophages. Examples of these viruses which infect macrophages are: HIV-1 in particular those strains possessing mutations in the V3 region of gp120, which mutations lead to increased binding to macrophages, HIV-2, Hanta viruses, for example Punmala virus, cytomegalovirus, respiratory syncytial virus, herpes simplex virus and filoviruses.
Examples of viruses which infect lymphocytes are: varicella zoster virus (VZV), herpes virus 6 (HHV-6), rabies virus, HIV-1, HTLV-II, HTLV-I, influenza C viruses because influenza C viruses bind, by way of the hemagglutinin-esterase fusion (HEF) protein, to N-acetyl-β-acetylneuraminic acid (Neu 5,9 Ac), which occurs preferentially on B lymphocytes and to a lesser extent, or not at all, on T lymphocytes; influenza C viruses possessing a mutation in nucleotide position 872 (which encodes position 284 of the HEF amino acid sequence), for example replacement of the threonine with isoleucine, because the HEF surface protein which possesses this mutation has a markedly stronger affinity for the N-acetyl-9-O-acetylneuraminic acid receptor than does the wild-type virus; influenza C virus HEF cleavage products which contain the structure for binding to N-acetyl-9-β-acetylneuraminic acid because this binding structure is defined by the catalytic triad serine 71, histidine 368 or 369 and aspartic acid 261; Epstein-Barr virus because EBV infects B cells, herpes simplex virus 2 because HSV-2 infects T cells, and measles virus.

### TS Ligands for Muscle Cells

Ligands that bind muscle cell surfaces include, for example, antibodies or antibody fragments which are directed against membrane structures of muscle cells, in particular of smooth muscle cells. Examples of antibodies of this nature are: antibody 10F3, antibodies against actin, antibodies against angiotensin II receptors or antibodies against receptors for growth factors or antibodies which are directed, for example, against EGF receptors, or against PDGF receptors, or against FGF receptors or antibodies against endothelin A receptors.

The TS ligands furthermore include all active substances which bind to membrane structures or membrane receptors on muscle cells. These ligands include, for example, growth factors, or their fragments or part sequences thereof, which bind to receptors which are expressed by smooth muscle cells, for example: PDGF; EGF; TGFβ; TGFα; FGF and endothelin A.

Within the meaning of this invention, the TS ligands also include envelope glycoproteins from those viruses which have a tropism for muscle cells. These viruses include cytomegalovirus, for example.

### TS ligands for hematopoietic cells

The TS ligands include antibodies or antibody fragments which are directed against receptors which are expressed on relatively undifferentiated blood cells. Antibodies of this nature have been described, for example, for the following receptors: stem cell factor receptor, IL-1 receptor (type I), IL-1 receptor (type II), IL-3 receptor α, IL-3 receptor β, IL-6 receptor and GM-CSF receptor.

The TS ligands furthermore also include monoclonal or polyclonal anti-bodies or antibody fragments which bind, by their constant domains, to Fc-γ receptors on immune cells. The TS ligands also include all substances which bind to membrane structures or membrane receptors on the surface of relatively undifferentiated blood cells. These ligands include, for example, growth factors, such as SCF, IL-1, IL-3, IL-6 and GM-CSF, or their fragments or part sequences thereof, which bind to receptors which are expressed by blood cells.

### TS ligands for synovial cells and inflammatory cells

These ligands include monoclonal or polyclonal antibodies or antibody fragments which bind, by their variable domains, to membrane structures of synovial cells or inflammatory cells. Examples of such membrane structures are vimentin, fibronectin and Fc receptors.
These ligands also include monoclonal or polyclonal antibodies or antibody fragments which bind, by their constant domains, to Fc receptors. These ligands furthermore include all active compounds which bind to membrane structures or membrane receptors on synovial cells. These include, for example, cytokines or growth factors, or their fragments or part sequences thereof, which bind to receptors which are expressed by synovial cells, for example IL-1-RA. TNFα, IL-4, IL-6, IL-10, IGF and TGFβ. These ligands furthermore include TS ligands whose essential constituent is terminal mannose, which binds to mannose 6-phosphate receptors on macrophages.

### TS ligands for cells that are infected with viruses

A TS ligand also may be an antibody or antibody fragment that is directed against a viral antigen which is expressed on the cell membrane of virus-infected cells. Antibodies of this nature have been described, for example, for cells which have been infected with the HBV, HCV, HSV, HPV, HIV, EBV and HTLV viruses.

### TS Ligands for Liver Cells and Other Tissue Cells.

The TS ligands include all substances which bind to membrane structures or membrane receptors on the surface of liver cells. These ligands include, for example, growth factors, such as cytokines, EGF, TGF, FGF or PDGF, or their fragments or part sequences thereof, which bind to receptors which are expressed by cells of this nature. These ligands furthermore include TS ligands which bind to cell membrane structures which are selective for particular tissues. Examples are:

**Table 1**

| | | |
|---|---|---|
| Membrane structure | TS ligand | Tissue cells |
| Asialoglycoprotein receptor | Asialoorosomucoid neoglycoprotein galactose | Liver cells |
| Transferrin receptor | Transferrin | Liver and other tissue cells |
| Insulin receptor | Insulin | Liver and other tissue cells |
| Mannose 6-phosphate receptor | Mannose | Macrophages in spleen, liver, lung and other tissues |
| Fc-γ receptors | Immunoglobulin G | reticuloendothelial system and other tissues |

These ligands and membrane structures are reviewed in Perales et al., Eur. J. Biochem. 226, 255 (1994).
Within the meaning of the invention, the TS ligands, include, in particular, envelope glycoproteins from viruses which have a tropism for selective cells, for example glycoproteins from respiratory syncytial virus for bronchial epithelial cells, glycoproteins from hepatitis C virus, filoviruses, hepatitis B virus and hepatitis D virus for liver cells. For example, liver cells bind the Marburg virus by way of the asialoglycoprotein receptor or liver cells bind, preferentially by way of the asialoglycoprotein receptor, to the preS2 and preS1 domains of HBV. Another example are glycoproteins from hepatitits B virus for liver sinusoidal cells., because HBV is bound by way of fibronectin.

### TS ligands for glia cells

These ligands include antibodies or antibody fragments which are directed against membrane structures of glia cells, as have been reported, for example, by Mirsky et al., Coakham et al. and McKeever et al. These membrane structures furthermore include neural adhesion molecules such as N-CAM, in particular its polypeptide C chain.

These ligands also include all active compounds which bind to membrane structures or membrane receptors on glia cells. For example, these ligands include substances which carry mannose in the terminal position and which bind to the mannose 6-phosphate receptor, insulin and insulin-like growth factor and PDGF, and those fragments of these growth factors which bind to the relevant membrane receptors.

Within the meaning of the invention, the TS ligands include, in particular, envelope glycoproteins from those viruses which have a tropism for glia cells. Examples of these viruses are HIV-1 subtype JRF 1 and herpes simplex virus I.

### TS ligands for leukemia cells

These ligands include antibodies or antibody fragments which are directed against membrane structures on leukemia cells. A large number of monoclonal antibodies of this nature have already been described for diagnostic and therapeutic methods (reviews in Kristensen, Danish Medical Bulletin 41, 52 (1994); Schranz, Therapia Hungarica 38, 3 (1990); Drexler et al., Leuk, Res. 10, 279 (1986); Naeim, Dis. Markers 7, 1 (1989); Stickney et al., Curr.. Opin. Oncol, 4, 847 (1992); Drexler et al., Blut 57, 327 (1988); Freedman et al., Cancer Invest, 9, 69 (1991)). The following monoclonal antibodies, or their antigen-binding antibody fragments, are suitable for use as ligands, depending on the type of leukemia: (1) for AML cells, membrane antigens CD13, CD14, CD15, CD33, CAMAL, sialosyl-Le; (2)for B-CLL membrane antigens CD5, CD1c, CD23, idiotypes and isotypes of the membrane immunoglobulins; (3) for T-CLL membrane antigens CD33, M38, IL-2 receptors, T cell receptors; (4) for ALL membrane antigens CALLA, CD19, non-Hodgkin's lymphoma.

The TS ligands furthermore include all active compounds which bind to membrane structures or membrane receptors of leukemia cells. These ligands include, for example, growth factors, or their fragments or part sequences thereof, which bind to receptors which are expressed by leukemia cells.

Growth factors of this nature have already been described (reviews in Cross et al., Cell 64, 271 (1991); Aulitzky et al., Drugs 48, 667 (1994); Moore, Clin. Cancer Res 1, 3 (1995); and Van Kooten et al., Leuk. Lymph. 12, 27 1993)). Examples are: IFNα in the case of non-Hodgkin's lymphomas; IL-2, particularly in the case of T cell leukemias; FGF in the case of T cell, monocytic, myeloid, erythroid and megakaryoblastic leukemias; TGFβ in the case of leukemias and retinoids, for example retinoic acid, in the case of acute promyelocytic leukemia.

### TS ligands for tumor cells

These ligands include antibodies and fragments of these antibodies which are directed against membrane structures on tumor cells. Antibodies of this nature have been reviewed, for example, by Sedlacek et al., Contrib. to Oncol. 32, Karger Verlag, Munich (1988) and Contrib. to Oncol. 43, Karger Verlag, Munich (1992).

Other examples are antibodies against: sialyl Lewis, peptides on tumors which are recognized by T cells, proteins which are expressed by oncogenes, gangliosides such as GD3, GD2, GM2, 9-O-acetyl GD3 and Fucosyl GM1, blood group antigens and their precursors, antigens on polymorphic epithelial mucin and antigens on heat shock proteins.

### THE LINKER

The choice of the linker depends on the chemical nature of the TS ligand and the GS ligand and on the method used to connect the TS ligand and the GS ligand to each other by way of the linker. If the ligands are peptides or proteins, a peptide or protein is preferably used as the linker and the linker is preferably connected to the TS ligand and the GS ligand by way of a peptide bond. TS ligand-linker-GS ligand or TS ligand-GS ligand-linker molecules of this nature are preferably prepared as fusion proteins using recombinant DNA technology. Generally, an advantageous linker will be an endogenously occurring substance or resemble an endogenous substance so as to limit its immunogenicity.

If the TS ligand is not a peptide or protein, the linker, in its simplest form, is then a structure which connects the TS ligand to the GS ligand. Structures of this nature result from the different chemical conjugation methods which are used to bond molecules to amino groups, hydroxyl groups, SH groups, carboxyl groups or aldehyde groups in proteins (for a review of the methods, see Sedlacek et al., Contrib. to Oncol. 32, 42-49 and 81-85, Karger Verlag, Munich (1988)).

The linker and the GS ligand can also themselves in each case be a peptide or protein. In this case, the connection between the two is preferably effected by way of a peptide bond and the connection to the linker by using one of the chemical conjugation methods. This applies, in particular, in the case of embodiment c) of the invention.

The choice of the linker also depends on the nature of the gene construct which is bound by the GS ligand. If the gene construct is a naked RNA or a. naked DNA, either alone or in a complex with a nonviral carrier, the linker is preferably a molecule having fusogenic properties. These fusogenic properties facilitate the passage of the gene construct through the cell membrane and out of the lysosomes into the cytoplasm.

If the gene construct is a virus, then a molecule having fusogenic properties can be selected as the linker. Within the meaning of this invention, use is preferably to be made of a linker having fusogenic properties. The fusogenic properties of the linker may compensate for the impairment of the fusogenic properties of the coat proteins of the virus, which is due to the GS ligand being bonded to the virus, or augment the fusogenic properties of the coat proteins of the virus.

Within the meaning of this invention, viral or bacterial peptides or proteins as well as synthetic peptides (for example those which form α-helices in the acid environment of the endosome) are used as linkers having fusogenic properties. Examples of molecules having fusogenic properties are: peptides which contain the translocation domain (domain III) of Pseudomonas exotoxin A (Wels et al., Cancer Res. 52, 6310 (1992); Fominaga et al., J. Biol. Chem. 271, 10560 (1996)), peptides which contain the peptide GLFEALLELLESLWELLLEA (SEQ ID No.: 1)
(Gottschalk et al., Gene Ther. 3, 448 (1996)), peptides which contain the peptide AALAEA[LAEA]₄LAAAAGC (SEQ ID No.: 2)) (Wang et al., Technol. Advances in Vector Syst. For Gene Ther., May 6-7, 1996, Coronado, IBC Conference), peptides which contain the peptide FAGVVLAGAALGVAAAAQI (SEQ ID No.: 3) of the measles virus fusion protein (Yeagle et al., Biochem. Biophys. Acta 1065, 49 (1991)), peptides which contain the peptide GLFGAIAGFIEGGWWGMIDG (SEQ ID No.: 4) of the influenza A HA2 protein (Lüneberg et al., J. Biol. Chem. 270, 27606 (1995)), peptides which contain the peptide GLFGAIAGFIENGWEGMIDGGLFGAIAGFIENGWEGMIDG (SEQ ID No. 5) (Burger et al., Biochem. 30, 11173 (1991) or the peptide GLFGAIAGFIE; (SEQ ID No.: 6), ALFGAIAGFIE; (SEQ ID No.: 7), LFLGAIAGFIE; (SEQ ID No.: 8), LLLGAIAGFIE; (SEQ ID No.: 9), LILGAIAGFIE; (SEQ ID No.: 10), GIFGAIAGFIE; (SEQ ID No.: 11), GLLGAIAGFIE; (SEQ ID No.: 12), GLFAAIAGFIE; (SEQ ID No.: 13), GLFEAIAGFIE; (SEQ ID No.: 14), GLFGAMAGFIE; (SEQ ID No.: 15) GLFGAIAGLIE (SEQ ID No.: 16) or the peptide
GLFGAIAGFIV (SEQ ID No.: 17) (Steinhauer et al., J. Virol. 69, 6643 (1995)) or the peptide GLFEAIAEFIEGGWEGLIEG (SEQ ID No.: 18) or the peptide GLLEALAELLEGGWEGLLEG (SEQ ID No.: 19) (Ishiguro et al., Biochem. 32 9792 (1993)).

Within the context of the present invention, use is furthermore made of proteins from viruses which possess fusogenic properties. A number of viruses possess fusogenic coat proteins, with examples being paramyxoviruses, retroviruses and herpesviruses (Gaudin et al., J. Gen. Virol. 76, 1541 (1995)). A number of viruses also possess glycoproteins which are responsible both for virus adherence and subsequent cell membrane fusion (Gaudin et al., J. Gen. Virol. 76, 1541 (1995)). Proteins of this nature are formed, for example, by alphaviruses, rhabdoviruses and orthomyxoviruses.

Viral fusogenic proteins within the meaning of the invention have been reviewed by Hughson, Curr. Biol. 5, 265 (1995); Hoekstra, J. Bioenergetics Biomembranes 22, 675 (1990); White, Ann. Rev. Physiol. 52, 675 (1990)). Examples of fusogenic proteins within the meaning of this invention are: the hemagglutinin of influenza A or influenza B viruses, in particular the HA2 component, the M2 protein of influenza A viruses, employed either alone or in combination (Ohuchi et al., J. Virol. 68, 920 (1994)) with the influenza hemagglutinin or with mutants of influenza A neuraminidase which lack enzyme activity but which nevertheless bring about hemagglutination, peptide analogs of influenza virus hemagglutinin, the HEF protein of influenza C virus the fusion activity of the HEF protein is activated by cleavage of the HEF0 into the subunits HEF1 and HEF2, the transmembrane glycoprotein of filoviruses, for example of Marburg virus and of Ebola virus, the transmembrane glycoprotein of rabies virus, the transmembrane glycoprotein (G) of vesicular stomatitis virus, the fusion protein of HIV virus, in particular the gp41 component and fusogenic components thereof, the fusion protein of Sendai virus, in particular the 33 amino terminal amino acids of the F1 component, the transmembrane glycoprotein of Semliki Forest virus, in particular the E1 component, the transmembrane glycoprotein of tickborne encephalitis virus, the fusion protein of human respiratory syncytial virus (RSV) (in particular the gp37 component), the fusion protein (S protein) of hepatitis B virus, the fusion protein of measles virus, the fusion protein of Newcastle disease virus, the fusion protein of visna virus, the fusion protein of murine leukemia virus (in particular p15E), the fusion protein of HTL virus (in particular gp21) and the fusion protein of simian immunodeficiency virus (SIV).

Viral fusogenic proteins are obtained either by dissolving the coat proteins from a viral enrichment with the aid of detergents (such as β-D-octylglucopyranoside) and separating them off by centrifugation, as reviewed by Mannio et al., BioTechniques 6, 682 (1988)) or else by means of molecular biological methods which are known to the skilled person. Examples of the preparation of fusogenic proteins have been described, for example, for influenza hemagglutinin, fusogenic fragments of influenza hemagglutinin, the M2 protein of influenza B, the HEF protein of influenza C, the transmembrane glycoprotein of filoviruses, for example Marburg virus and Ebola virus, the transmembrane glycoprotein of rabies virus, the transmembrane glycoprotein of vesicular stomatitis virus, the transmembrane glycoprotein of Semliki forest virus, the transmembrane glycoprotein of tickborne encephalitis virus and the transmembrane glycoprotein of HIV-1 virus.

### THE GENE CONSTRUCT-SPECIFIC LIGAND

Within the context of the present invention, the GS ligand is a structure which bonds directly or indirectly to the gene construct and contains an entire antibody molecule or an epitope-binding fragment of an antibody. Murine monoclonal antibodies are preferably employed in humanized form to limit their immunogeneity. This humanization is effected, as already described in the section entitled "Description of the TS ligand", in the manner depicted by Winter et al. (Nature 349, 293 (1991)) and Hoogenboom et al. (Rev. Tr. Transfas. Hemobiol. 36, 19 (1993)). Antibody fragments and recombinant Fv fragments are prepared in accordance with the state of the art and as already described in the section entitled "Description of the TS ligand".

Whether a bivalent or a monovalent fragment is used depends on the choice of antibody specificity and gene construct. A monovalent antibody fragment is preferred if the chosen antibody impairs the fusion activity of the coat protein of a viral gene construct (as described, for example, by Ubol et al., J. Virol. 69 1990 (1995).

The specificity of the antibody depends on the nature of the gene construct which is used. If the gene construct is a naked RNA or a naked DNA, either alone or in a complex with a nonviral carrier, one of the novel embodiments of this invention is that the specificity of the antibody is directed against those epitopes which have been introduced into the DNA.

Epitopes of this nature can be generated by one or more modifications of the DNA, with or without the introduction of a foreign group (xenogenic substance). Examples of this include crosslinking the DNA with cisplatin, alkylating the N⁷ of guanine with an alkylating agent such as nitrogen mustard, melphalan or chlorambucil, intercalating an anthracycline, such as doxorubicin or daunomycin, into the double helix of the DNA.

Examples of monoclonal antibodies with binding specificity against a modified DNA epitope include antibodies directed against methylated DNA, O⁶-ethyldeoxyguanosine (after treating the DNA with ethylnitrosourea), N⁷-ethylguanine, N⁵-methyl-N⁵-formyl-2,5,6-triamino-4-hydroxypyrimidine,O⁶-methyl-2'-deoxyguanosine, O⁶-ethyl-2'-deoxyguanosine, O⁶-n-butyl-2'-deoxyguanosine, O⁶-isopropyl-2'-deoxyguanosine, O⁴-methyl-2'-deoxythymidine, O⁴-ethyl-2'-deoxythymidine, addition products of melphalan and DNA and anthracyclines. Some useful epitopes in this context are those created in DNA by methylation of the DNA during DNA metabolism.

E. coli strains are known to methylate plasmid DNA that has been introduced into the bacterium. The methylation occurs at the N⁶ position of adenine (Winnacker, From Genes to Clones, page 18/19, VCH Publisher, Weinheim (1987)). Bacteria possess the enzyme DNA adenine methylase, which specifically methylates adenines at the N⁶ position during replication (Hattman et al., J. Mol. Biol. 126, 367 (1978)). Consequently, this invention relates, in particular, to the use of monoclonal antibodies against methylated DNA and more particularly against methylated N⁶ of adenine in the novel ligand system.

If the gene construct is complexed with a nonviral carrier, another particular embodiment of this invention is that the specificity of the antibody is directed against an epitope on the carrier. These carriers include cationic polymers, peptides, proteins, polyamines or cationic lipids, such as cationic lipids and phospholipids. Examples of antibodies against carriers of this nature are antibodies against spermidine, spermine, putrescine, polylysine, albumin and phospholipid.

If the gene construct is a virus, the specificity of the antibody against one or more identical or different epitopes of a viral coat protein. Since the linker in the ligand system employed is preferably a fusogenic peptide or protein, use also can be made of antibodies which impair the cell adhesion and/or the fusogenic activity of the virus by binding to the coat protein.
Examples of antibodies against coat proteins of viruses which can be used as vectors are antibodies against murine leukemia virus, in particular against the coat proteins gp70 and p15, HIV virus, adenovirus, herpes simplex virus, in particular against glycoprotein B, glycoprotein H, glycoprotein L and glycoprotein D of this virus, cytomegalovirus, in particular against glycoprotein B (gpB), minute virus of mice, adenoassociated virus, in particular against the cap and rep proteins, *Sindbis* virus, in particular against envelope protein E2 or E and vaccinia virus.

In another advantageous embodiment of the invention, the GS ligand is the part of an Fc receptor which is outside the cell. One of the previously mentioned antibodies, which binds directly or indirectly, by its antigen-binding part, to the gene construct, binds to this Fc receptor by way of its Fc part.

In another advantageous embodiment, the GS ligand is a cationic structural unit, such as a cationic amino acid, a cationic peptide or protein or a biogenic amine, which is able to complex with the gene construct. Examples of these cationic structural units include, lysine, polylysine, arginine, polyarginine, histidine, polyhistidine, peptides that contain at least 1 lysine, 1 arginine and/or 1 histidine and polyamines such as cadaverine, spermidine, spermine, agmatine or putrescine.

In another advantageous embodiment, the GS ligand is a receptor for the coat protein of the virus which harbors the transgene. Receptors of this nature have, for example, been described for the following viruses: HIV concerning the CD4 molecule (soluble or native) and galactosylceramide, HBV concerning the IL-6 receptor and annexine or apolipoprotein, HTLV concerning the IL-2 receptor (the β and γ chains), measles virus concerning the CD46 molecule, Friend leukemia virus concerning the erythropoietin receptor, varicella Zoster concerning the Fc fragment of human immunoglobulin G, Sendai virus concerning the glycophorin, influenza C virus concerning N-acetyl-9-acetamido-9-deoxyneuraminic acid and 9-O-acetyl-N-acetylneuraminic acid, foot and mouth disease virus concerning integrin αVβ3, EBV concerning Complement receptor 2 (CD21) and herpes simplex virus concerning the 275 kDa marnose 6-phosphate receptor or the 46 kDa mannose 6-phosphate receptor.

### THE LINKER AS A COMPLEX ("CONNECTOR") OF AT LEAST Two MOLECULES

Within the meaning of special embodiment c) of the invention, the connector is a special kind of linker that comprises at least two molecules or components. One of the molecules or components of the connector binds to at least one gene construct-specific ligand and another molecule or component of the connector binds to at least one target cell-specific ligand. The complex further advantageously comprises at least one other "optional linker". The optional linker may be a fusiogenic peptide. In case of more than one linker the optional linker may be chemically identical or different. The fusogenic peptide facilitates entry of nucleic acid into the target cell. Another optional linker in this context may be a signal producing substance such as a radioactive isotope, to allow quantitation of complexes that enter cells.

An advantageous example of a connector is the hinge region of an antibody, by way of which the two heavy chains of the antibody are connected to each other (Burbon, TIBS 15, 64, (1990); Oi et al., Nature 307, 136 (1984); Alt et al., Science 238, 1079 (1987); Lorenz, degree dissertation: Konstruktion und Expression von rek. Antikörper-Enzym-Hybridmolekülen für die Tumortherapie [Construction and expression of rec. antibody/enzyme hybrid molecules for tumor therapy], Faculty of Human Medicine, Marburg University (1991)).

A novel arrangement of the hinge region is depicted, for example, in diagram c1) in Figure 2. The hinge region is preferably connected to the linkers and to the GS and TS ligands by way of peptide bonds, in the form of a fusion protein, which fusion protein is prepared using recombinant DNA techniques.

Another example of a connector is the Gal80 protein (Leuther et al., Science 256, 1333 (1992)) in combination with the Gal80-binding domain of Gal4 (Leuther et al., Science 256, 1333 (1992)) in accordance with diagram c2) in Figure 3.

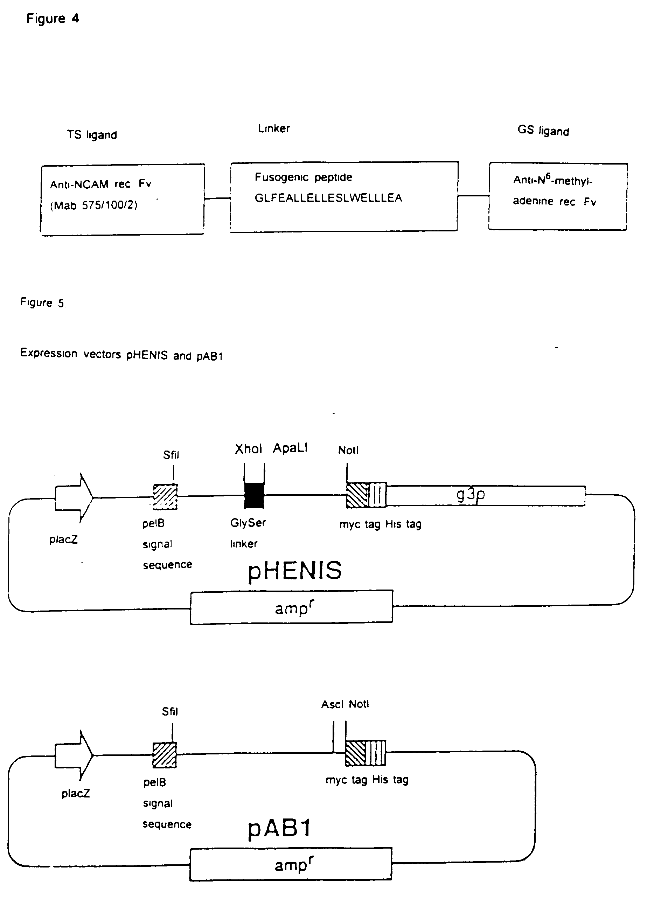

The following examples are presented by way of illustration and not by way of limitation and indicate construction of a multifunctional system as depicted in Figure 4.

### EXAMPLE ONE

### Preparation of a TS ligand

The hybridoma of the anti-NCAM monoclonal antibody 575/100/2 is used as the starting material for the TS ligand (Jaques et al., Cancer 72, 418 (1993)). Approximately 10⁷ cells of this hybridoma are separated by centrifugation and the mRNA is extracted from these cells using the Pharmacia mRNA extraction kit. This mRNA is then transcribed into cDNA by reverse transcription using a cDNA synthesis kit and random hexaoligonucleotides (from Pharmacia). This cDNA serves as the starting material for amplifying the variable heavy chain or the variable light chain of the immunoglobulin by means of the polymerase chain reaction (Saiki et al., Science 230, 1350 (1985)) using specific primers (Clackson et al., Nature 352, 624 (1991)). At the same time, the primers introduce restriction cleavage sites for cloning the fragments into the bacterial expression vector pHENIS (which is derived from pHEN1; Hoogenboom et al., Nucl. Acids Res. 19, 4133 (1991); see Figure 5). This vector contains a pelB signal sequence for periplasmic secretion, a myc tag for detection with the monoclonal antibody 9E10, a histidine tag for purification by means of immobilized metal affinity chromatography (IMAC), as well as a cloning region for the heavy and light chains and a short sequence which encodes a 14 amino acid-long glycine-serine linker. In addition, fusion with the g3β protein is effected for the purpose of displaying on the surface of bacteriophages. The heavy and light chains are digested with the appropriate restriction enzymes (VH with SfiI and ShoI; VL with ApaLI and NotI) and cloned one after the other into the vector. This results in a recombinant single-chain Fv fragment comprising the variable heavy chain and light chain, which are covalently linked by way of a short peptide sequence.

### EXAMPLE TWO

### Preparation of a GS ligand

Recombinant antibodies possessing specificity for N⁶-methyladenine (from Sigma) are selected from native or semisynthetic antibody libraries (Nissim et al., EMBO J. 13, 692 (1994)), as described, by biopanning on N⁶-methyladenine-BSA or N⁶-methyladenine-thyroglobulin conjugates (Beiser et al., Methods Enzymol. XII, 889 (1968)). Positive antibody fragments are identified by ELISA in antigen-coated microtiter plates (Nissim et al., EMBO J. 13, 692 (1994)). Antibodies from these libraries are already in the desired single-chain Fv format and can be employed directly for subsequent clonings.

### EXAMPLE THREE

### Preparation of a linker

A fusogenic peptide having the amino acid sequence GLFEALLELLESLWELLLEA (SEQ ID No.: 1, Gottschalk et al., 1996) is used as the linker. The DNA encoding this peptide is prepared as a double-stranded synthetic oligonucleotide, with suitable restriction cleavage sites (AscI and XbaI) being coupled onto the ends. For this, the two synthetic oligonucleotides
O1 (5'GGCCGCAGGCTTATTTGAGGCCCTTCTGGAATTGCTAGAGAGCCTC-TGGGAATTGCTTCTGGAGGCAT, SEQ ID No.: 20)
and O2 (5'CTAGATGCCTCCAGAAGCAATTCCCAGAGGCTCTCTAGCAAT-TCCAGAAGGGCCTCAAATAAGCCTG, SEQ ID No.: 21) are phosphorylated using T4 polynucleotide kinase (from Gibco) in accordance with the manufacturer's instructions, heated at 80°C for 5 min and then slowly cooled down to room temperature. This double-stranded DNA fragment is used directly for subsequent clonings.

### EXAMPLE FOUR

### Preparation of a Multifunctional Ligand

The complete ligand system is prepared in expression vector pAB1 (which is constructed in a similar manner to pHENIS but does not include fusion with g3p; see Fig. 5) in the form of a 3-fragment cloning. The anti-NCAM single-chain Fv fragment (TS ligand) which was cut with the restriction enzymes SfiI and NotI, the linker, which contains the cloning sites NotI and XbaI, and the anti-N⁶-methyladenine single-chain Fv fragment (GS ligand) are used as the starting material. For the cloning, the GS fragment is reamplified using primers which insert the restriction cleavage sites XbaI and AscI at the N terminus and C terminus, respectively. These fragments are cloned into the pAB1 vector, which has been cut with the restriction enzymes SfiI and AscI. The construct is transformed into bacterial strain TG1. Expression of the ligand system is regulated by way of the bacterial lacZ promoter and is induced by adding isopropyl-β-D-thiogalactoside (IPTG) (as described in McCafferty et a., Appl. Biochem. Biotech. 47, 157 (1994)). The expressed protein is purified from periplasmic preparations by means of IMAC in accordance with the method of Griffiths et al., EMBO J. 13, 3245 (1994). The total protein has a molecular weight of approx. 55,000 daltons and is present as a monomer.

### EXAMPLE FIVE

### Testing the Operation of the Multifunctional Ligand

NCAM-expressing tumor cells (small cell bronchial carcinoma) are multiplied in cell culture, using the cell culture technique known to the skilled person, and isolated. DNA which is methylated on the N⁶ of adenine is prepared by multiplying a plasmid (which contains the structural gene for β-glucuronidase, see Patent Application WO96/06940) in E. coli.
The multifunctional ligand system is mixed with the plasmid DNA in a molar ratio of 20:1 and the mixture is incubated at 37°C for 30 min. Binding to the plasmid DNA is checked by ELISA. The complex comprising the multi-functional ligand and the plasmid is mixed with the tumor cells in a ratio of 10:1 and the whole is incubated at 37°C for 1 hour. A portion of the tumor cells are washed. The binding of the complexes to these tumor cells is checked by means of immunofluorescence. The remaining tumor cells are incubated for a further 24 hours. Successful uptake of the complexes into the cell, linker-mediated release from the endosomes, and transcription and expression of the effector gene are determined by detecting the enzymic activity of β-glucuronidase in the culture medium using 4-methylumbelliferyl-β-glucuronide as the substrate.

## Claims

1. A multifunctional ligand system for target cell-specific transfer of a nucleotide sequence, comprising at least one target cell-specific ligand, at least one gene construct-specific ligand that comprises an antibody or an antibody portion, and a linker that links the two ligands, wherein said system is not immunogenic.

2. A ligand system as claimed in claim 1, further comprising a connector that connects said two ligands together and wherein the connector comprises two linkers.

3. A ligand system as claimed in claim 1 or 2, wherein said target cell-specific ligand binds to the surface of a target cell.

4. A ligand system as claimed in any of claims 1 - 3, wherein said target cell-specific ligand is selected from the group consisting of a growth factor, a cytokine, an interferon, a tumor necrosis factor, a chemokine, a peptide hormone, an angiotensin, a kinin, a histamine, a steroid hormone, an adhesion molecule, a VDL receptor ligand, an LDL receptor ligand, an oxidized LDL receptor ligand, an LDL-related receptor protein ligand, an IgG Fc receptor ligand, an 88 kDa glycoprotein receptor ligand, an acetylated LDL receptor ligand, a megalin ligand, and a vitamin.

5. A ligand system as claimed in any of claims 1 - 3, wherein said target cell-specific ligand is an antibody or antibody fragment which binds to the target cell.

6. A ligand system as claimed in claim 5, wherein said antibody fragment is selected from the group consisting of an F(ab)₂ fragment, an Fab fragment, a double-chain Fv fragment, a single-chain Fv fragment and an Fc fragment.

7. A ligand system as c]aimed in any of claims 1 - 3, wherein said target cell-specific ligand is an extra-cellular region of an Fc receptor that is recognized by an antibody Fc fragment.

8. A ligand system as claimed in any of claims 5 - 7, wherein said antibody, or antibody fragment is at least partially of human origin.

9. A ligand system as claimed in any of claims 1 - 8, wherein said gene construct-specific ligand is selected from the group consisting of an antibody, an antibody fragment, a cationic structural unit, and a receptor of a coat protein of a virus.

10. A ligand system as claimed in claim 9, wherein said antibody fragment is selected from the group consisting of an F(ab)₂ fragment, an Fab fragment, a double-chain Fv fragment and a single-chain Fv fragment.

11. A ligand system as claimed in any of claims 1 - 8, wherein said gene construct-specific ligand is an extra-cellular region of an Fc receptor that is recognized by an antibody Fc fragment.

12. A ligand system as claimed in any of claims 1 - 11, wherein said antibody or said antibody fragment is at least partially of human origin.

13. A ligand system as claimed in any of claims 1 - 12, wherein said antibody or said antibody fragment binds specifically to an epitope of nucleic acid that has been introduced by bonding a xenogenic substances to the nucleic acid, methylating the nucleic acid, or alkylating the nucleic acid.

14. A ligand system as claimed in any of claims 1 - 13, wherein said antibody or said antibody fragment binds an epitope on a nonviral carrier.

15. A ligand system as claimed in claim 14, wherein said nonviral carrier is selected from the group consisting of a cationic polymer, peptide, protein, biogenic amine, polyamine, lipid and phospholipid.

16. A ligand system as claimed in any of claims 1 - 15, wherein said antibody or said antibody fragment binds an epitope of a viral coat protein.

17. A ligand system as claimed in claim 16, wherein said virus is selected from the group consisting of murine leukemia virus, HIV, adenovirus, herpes simples virus, cytomegalovirus, minute virus of mice, adenoassociated virus, Sindbis virus and vaccinia virus.

18. A ligand system as claimed in claim 9, wherein said cationic structural unit comprises, alone or in combination, at least 1 lysine residue, at least 1 arginine, at least 1 histidine and at least 1 polyamine.

19. A ligand system as claimed in any of claims 2 - 18, wherein said connector comprises a moiety from a covalent attachment to a functional residue, the functional residue selected from the group consisting of an amino residue, a hydroxyl residue, a SH residue, a carboxyl residue and an aldehyde residue.

20. A ligand system as claimed in any of claims 2 - 19 wherein at least one of said linkers is a fusogenic substance.

21. A ligand system as claimed in claim 20, wherein said fusogenic substance is selected from the group consisting of a synthetic fasogenic peptide, a bacterial fusogenic peptide or protein, and a fusion product between a peptide or protein and a virus.

22. A ligand system as claimed in any of claims 2 - 21, wherein said connector comprises the hinge region of an antibody or the Gal80 protein in combination with the Gal4 protein.

23. A ligand system as claimed in claim 1 comprising:
(a) a target cell-specific ligand comprising an anti-NCAM recombinant single-chain Fv fragment, wherein the variable heavy chain and light chain of the Fv fragment are covalently linked by way of a short peptide sequence;
(b) a linker comprising a fusogenic peptide having the sequence GLFEALLELLESLWELLLEA (SEQ ID No.: 1); and
(c) a gene construct-specific ligand comprising a recombinant antibody for N⁶-methyladenine.

24. A ligand system as claimed in claim 23, further comprising a gene construct.

25. A ligand system as c]aimed in claim 24, wherein said gene construct is selected from the group consisting of a naked RNA, a plasmid, a naked nucleic acid or plasmid combined with a cationic polymer, peptide, protein, or lipid, and a virus.

26. Use of the ligand system claimed in any of claims 1 - 25 for the manufacture of a pharmaceutical to prevent or treat a skin disease, mucous membrane disease, nervous system disease, internal organ disease, blood coagulation disease, hematopoietic system disease, immune system disease, musculature disease, and sustentacular tissue or joint disease.
